# EUROPEAN PATENT SPECIFICATION

(11) **EP 1 641 475 B1**
(45) Date of publication and mention of the grant of the patent: **15.06.2011**
(21) Application number: 04743212.5
(22) Date of filing: 02.07.2004
(51) Int. Cl.: A61K 35/74, A61P 3/06

(54) **LIPIDS FROM METHANOTROPHIC BACTERIA FOR CHOLESTEROL REDUCTION**
LIPIDE AUS METHANOTROPHISCHEN BAKTERIEN ZUR SENKUNG VON CHOLESTERIN
LIPIDES EXTRAITS DE BACTERIES METHANOTROPHES DESTINES A LA REDUCTION DU CHOLESTEROL

(30) Priority: 04.07.2003 GB 0315783
(43) Date of publication of application: 05.04.2006
(73) Proprietor: Statoil ASA, 4035 Stavanger (NO)
(72) Inventor: MÜLLER, Hanne, 0280 Oslo (NO); SKREDE, Anders, 1430 Ås (NO); KLEPPE, Gunnar, 4044 Hafrsfjord (NO)
(74) Representative: Golding, Louise Ann
(86) International application number: PCT/GB2004/002866
(87) International publication number: WO 2005/004888

(56) References cited:
- EP-A- 0 404 300
- WO-A-01/49277
- WO-A-01/60974
- WO-A2-03/072133
- GB-A- 1 319 114
- DATABASE MEDLINE [Online] US NATIONAL LIBRARY OF MEDICINE (NLM), BETHESDA, MD, US; November 1975 (1975-11), WEAVER T L ET AL: "Whole-cell and membrane lipids of the methylotrophic bacterium Methylosinus trichosporium." XP002302098 Database accession no. NLM810477 & JOURNAL OF BACTERIOLOGY. NOV 1975, vol. 124, no. 2, November 1975 (1975-11), pages 602-605, ISSN: 0021-9193
- DATABASE BIOSIS [Online] BIOSCIENCES INFORMATION SERVICE, PHILADELPHIA, PA, US; 1991, BOWAN J P ET AL: "PHOSPHOLIPID FATTY ACID AND LIPOPOLYSACCHARIDE FATTY ACID SIGNATURE LIPIDS IN METHANE-UTILIZING BACTERIA" XP002302099 Database accession no. PREV199191111571 & FEMS MICROBIOLOGY ECOLOGY, vol. 85, no. 1, 1991, pages 15-22, ISSN: 0168-6496
- DATABASE BIOSIS [Online] BIOSCIENCES INFORMATION SERVICE, PHILADELPHIA, PA, US; 1993, PELTOLA PETRI ET AL: "Effect of copper on membrane lipids and on methane monooxygenase activity of Methylococcus capsulatus (Bath)" XP002302100 Database accession no. PREV199396092064 & ARCHIVES OF MICROBIOLOGY, vol. 159, no. 6, 1993, pages 521-525, ISSN: 0302-8933
- DATABASE WPI Week 199549, Derwent Publications Ltd., London, GB; Class D16, AN 1995-381113 GRIGORYAN A N; KAZANTSEV YU E; SILANTEV L V: 'METHOD OF OBTAINING LIPIDS' & SU 1 040 797 A1 (VSESOYUZNYJ NII BIOSINTEZA BEL [SU]) 27 May 1995
- DATABASE WPI Week 199443, Derwent Publications Ltd., London, GB; Class D16, AN 1994-348593 SULTANOVICH YURIJ A [SU]; KOLESNIK GALINA B [SU]; ROZHDESTVENSKAYA MARINA V [SU]; KRYUKOVA ELIZAVETA V [SU]; ZAJTSEV SERGEJ A [SU]: 'PROCESS FOR PREPARING ETHYL FATTY ACID ESTERS' & SU 1 822 411 A3 (MO T I PISHCHEVOJ PROMY [SU]) 15 June 1993

## Description

This invention relates to the use of microbial cells and extracts and derivatives thereof for the treatment of human or non-human vertebrate animal, especially mammalian, avian or piscine, subjects to modify lipid metabolization, in particular to reduce plasma cholesterol levels therein or to maintain low plasma cholesterol levels therein.

Overly high total plasma cholesterol levels in mammals are associated with increased risk of coronary heart disease. Those particularly at risk include the overweight, smokers, those with a poor diet (e.g. one rich in saturated fats), those who take inadequate exercise, and those suffering from stress. This is not a problem restricted to humans but is also one which occurs with pet animals and farmed animals.

For at risk individuals, as well as those tested and found to have unduly high plasma cholesterol levels, a variety of treatments have been prepared, e.g. changes in diet and habits, increased exercise, etc. However such treatments are not always easy to enforce and there remains a need for treatments which can be effective at reducing plasma cholesterol levels.

Aspects of microbial lipids from methanotrophic bacteria have been studied. For example, the phospholipid fatty acid and lipopolysaccahride fatty acid signature of lipids in certain methane-utilizing bacteria has been studied by Bowman et al. (FEMS Microbiology Ecology (1991), 85, pp.15-22).

Other aspects of microbial lipids are disclosed in the patent literature. Russian patent publication No. SU1040797-A1 (Proteins Biosynthesis Res. Inst.) discloses the production of lipids rich in palmitic and palmito-oleic acids from *Methylococcus* or *Methylomonas* type bacteria. Russian patent publication No. SU1822411-A3 (Mosc. Food Ind. Techn. Inst.) discloses the synthesis of ethyl esters of fatty acids using lipase, wherein the source of the lipase and fatty acids is *Methylococcus capsulatus* VKPM-1743.

No medical uses for microbial lipids are disclosed in the above-mentioned documents. Furthermore, the above-mentioned documents do not disclose foodstuffs or pharmaceutical or nutraceutical compositions comprising microbial lipids from methanotrophic bacteria.

International patent publication No. WO 03/072133-A2 (Norferm DA) discloses the use of a composition comprising biomass derived from a methanotrophic bacterium-containing culture as an immunostimulant. However, this document does not disclose the uses of microbial lipids as presently claimed. In particular, WO 03/072133-A2 is silent as to any pharmaceutical effects of a lipid component from methanotrophic bacteria.

In WO 01/60974 and WO 03/016460 there is described a method by which microorganisms may be cultured, with the primary intention of producing a protein-containing material which could be used as a foodstuff or as an additive therefor.

We have now surprisingly found that lipids produced by microbes (e.g. bacteria, yeast or fungi, in particular bacteria) when used as a major or minor part of the lipid intake in an animal's diet, serve to reduce the animal' s plasma cholesterol levels. Thus such microbial lipids may be used in a method of treatment to reduce plasma cholesterol, to maintain a reduced plasma cholesterol level, or to reduce the ratio of LDL to HDL cholesterol.

Thus viewed from one aspect the invention provides microbial lipids for oral administration for use as claimed in claims 1 to 13. It also provides the use of microbial lipids for the manufacture of a medicament for oral administration for use in the treatment of animal subjects to reduce plasma cholesterol, in particular LDL cholesterol, levels therein, or to reduce the ratio of LDL to HDL cholesterol in the plasma thereof, e.g. by increasing plasma HDL cholesterol levels.

It has also been surprisingly found that administration of the microbial lipids herein described increases an animal's plasma DHA (docosahexaenoic acid) levels.

In particular, analyses of blood plasma from mink as in Example 1 below surprisingly showed that plasma docosahexaenoic acid (22:6 n-3) increased with increasing level of microbial lipids. The average plasma levels of DHA following feeding of 0%, 17% and 67% microbial lipids (replacing soybean oil) were 5.99, 7.65, and 11.39 mol/100 mol fatty acids. This effect of modification of dietary lipids is highly significant. Neither soybean oil nor the microbial lipids used in the trial contain appreciable amounts of DHA. Thus, higher levels of DHA in the blood plasma of animals fed microbial lipids, compared with soybean oil, were not of dietary origin.

The microbial lipids, particularly the phospholipids and especially phosphatidylethanolamine, thus contribute to higher levels of DHA in blood plasma in the human or animal recipient. Interestingly, the highest level of DHA in the body occurs in phosphatidylethanolamirie, the most prominent phospholipid class in the microbial lipid used in the trial. The highest levels of DHA occur in neural tissues, brain and eyes, to a great extent associated with phosphatidylethanolamine. DHA is of great importance in neural tissue, and for reproduction as well as early postnatal life. Fish lipids are the greatest source of DHA in most human diets, and this is an important reason why an intake of fish lipids is so important in human nutrition. Recent published studies show that DHA (and also eicosapentaenic acid (EPA), e.g. 20:5 n-3) is beneficial to cardiovascular health, i.e. improved endothelial function, and lower blood pressure, platelet sensitivity, and serum triglyceride level in humans. The use of the microbial lipids according to the invention is thus beneficial to human and animal health due to increasing the amounts of available DHA in the metabolism.

Thus viewed from a further aspect the invention provides the use of microbial lipids produced by methanotrophic bacteria for the manufacture of a medicament for oral administration for use in the treatment of animal subjects for improving cardiovascular health by increasing plasma DHA.

Furthermore, it has been found that juvenile animals especially juvenile fish benefit from an immuno-protective effect brought on by administration of the microbial lipids herein described.

Lipids are the most suitable source of dietary energy in fish feed. It is well known that the n-3 highly unsaturated fatty acids, i.e. 20:5 n-3 and 22:6 n-3, are required to satisfy the requirement for essential fatty acids in fish. The high levels of DHA in neural tissue may be particularly importance in the feeding of fish larvae. In fish feed, fish oil is the main dietary source of these fatty acids. Fish oil is at present a limited resource, and insufficient fish oil supply is expected to limit expansion in aquaculture in the future. There is evidence that fish larvae have limited capacity to synthesise phospholipids. The use of the microbial lipids according to the invention, by virtue of their high contents of phospholipids, in particular phosphatidylethanolamine, is thus beneficial to fish health and survival, especially in the early (eg first feeding to juvenile) stages of life. This is also particularly due to the effect of increasing levels of DHA available in the metabolism.

The word animal as used herein is used to refer to humans and non-humans, particularly vertebrates, e.g. mammals, birds and fish, but also, less preferably shellfish. Use of the invention in relation to humans and other mammals as well as gallinaceous birds (e.g. domestic fowl) and farmed fish (e.g. salmon and trout) is particularly preferred. Use in relation to humans, non-human mammals and domestic fowl is especially preferred. Particularly preferred is use in relation to juvenile animals, especially juvenile fish.

By a microbial lipid is meant herein a lipid produced by a microbe, e.g. a bacteria, yeast or fungus.

Such lipids will generally be produced so as to constitute part of the microbe's internal or external membranes. Typically such lipids comprise phospholipids, e.g. phosphatidylglycerols, phosphatidylethanolamines, phosphatidylcholines, and cardiolipins. The preferred source of the microbial lipids used according to the invention are maj oratively phospholipids (i.e. the head group to which the fatty acid side chains are attached includes a phosphorus atom). The use of methanotrophic bacteria which are bacteria having membranes for nutrient gas fixation and which have a high phospholipid content and in particular a high phosphatidylethanolamine content (relative to total lipid content, e.g. a phospholipid content of at least 30% wt., preferably a phosphatidylethanolamine content of at least 30% wt. relative to total lipid content) is especially preferred, especially methylococcus bacteria. The use of methanotrophic bacteria in which phospholipids, and especially phosphatidylethanolamines, constitute at least 60% wt of the total lipid contact is especially preferred.

Quite surprisingly such microbial phospholipids have a marked cholesterol reducing effect despite the fact that the fatty acid side chains are predominantly saturated or monounsaturated (i.e. they are fatty acids of a type which would be expected to increase plasma cholesterol).

Interestingly, plasma triacylglycerol levels may also be reduced by administration of microbial lipids in accordance with the present invention and the reduction in triacylglycerols rather than or as well as an effect on cholesterol may be the aim of administration of the claimed products.

The fatty acid side chains in such lipids are generally C₁₄ to C₂₂ fatty acids, particularly C₁₆ fatty acids and in a preferred embodiment these are predominantly (e.g. >80% wt.) saturated or monounsaturated fatty acids, especially C_{16:0} and C_{16:1} fatty acids. The microbial lipid used according to the invention preferably contains phosphatidylethanolamines as the major lipid constituent, e.g. at least 50% wt., more preferably at least 65% wt.

The microbial lipids used according to the invention may if desired be separated from the other components of the microbes before use, e. g. from proteins, nucleic acids, etc. However, since the microbial lipids may be used as part of the dietary intake of the animal, i.e. to meet part of its nutritional needs, such lipid separation is not essential since the other components may also contribute to the animal's nutritional needs.

In general, it is preferred that the microbial lipid be used in the form of a lipid extract from a microbial biomass, e.g. a culture, prepared for example as described in WO 01/60974 and WO 03/016460. The homogenization step referred to in WO 01/60974 may be omitted and the biomass from the reactor may be subjected to a conventional lipid extraction technique, e.g. supercritical extraction or solvent extraction. Preferably such extraction will involve use of a polar organic solvent or solvent mixture, e.g. a mixture of an alcohol and a halocarbon, especially a methanol/chloroform mixture, particularly a 1:2 v/v methanol/chloroform mixture.

Microbial lipid extracts are chemically distinct from plant derived lipids in terms of their fatty acid profile as they are substantially free from polyunsaturated fatty acids. As a result they have increased storage stability. The microbial lipid extracts used according to the invention are preferably at least 80% wt. pure, especially at least 90% wt. pure, particularly at least 95% wt. pure, e.g. >99% wt. pure, and may be provided in a quantity of at least 10g, especially at least 50g, more especially at least 100g.

Following extraction from the microbial biomass, the lipid extract may if desired be purified or separated into lipid fractions (e.g. chromatographically). The phospholipid (-containing) fractions, and in particular the phosphatidylethanolamine (-containing) fractions, especially the fraction(s) containing phosphatidylethanolamines with C_{16:0} and/or C_{16:1} fatty acid side chains, are especially preferred for use according to the invention.

A preferred microbe-derived phospholipid for use according to the invention is a phosphatidylethanolamine, and particularly a phosphatidylethanolamine with C_{16:0} and/or C_{16:1} fatty acid side chains, preferably at least 80% wt. pure, especially at least 90% wt. pure, particularly at least 95% wt. pure, e.g. >99% wt. pure, and preferably in a quantity of at least 10g, especially at least 50g, more especially at least 100g, e.g. for use in medicine.

Such microbial lipid extracts may be used as ingredients in foodstuffs, e.g. as a total or partial replacement for fats contained therein, or as an additional lipid component. Typical such foodstuffs include margarines and other spreads, mayonnaise and other salad dressings, yoghurts, creams (including non-dairy creams etc.), cheeses and cooking oils and fats.

Alternatively, the microbially derived lipids may be administered in pharmaceutical or nutraceutical form, e.g. formulated as solutions, suspensions, emulsions micro-emulsions, vesicles or micro-encapsulated forms, or in capsules, etc. Conventional pharmaceutical carriers and excipients, such as stabilizers, emulsifiers etc. and conventional formulation techniques may be used. Where the product is in dosage unit form, the dose unit preferably contains 100 to 1500 mg, especially 300 to 700 mg, particularly 400 to 600 mg. Formulation in capsule form is especially preferred, e.g. using gelatin capsule cases. A daily dose would typically be 0.02 to 0.5 g/kg bodyweight, preferably 0.05 to 0.25 g/kg bodyweight.

Thus viewed from a further aspect the invention provides a foodstuff containing a methanotrophic bacterial lipid extract (preferably providing at least 5% wt. of the total lipid content of the foodstuff, more preferably at least 10% wt, especially at least 25% wt.) and a further nutrient component, preferably of non-microbial origin.

Viewed from a still further aspect the invention also provides a pharmaceutical or nutraceutical composition comprising a methanotrophic bacterial lipid extract together with a pharmaceutically acceptable carrier or excipient.

The animal recipient of the microbial lipids according to the invention will preferably be a mammal or bird found to have elevated plasma cholesterol levels or considered to be at risk of elevated plasma cholesterol levels, e.g. due to its weight, diet, habits or living conditions. Such animals include in particular humans and fish; however the invention is also applicable to pet animals (e.g. dogs, cats, rabbits, guinea pigs, etc.) and to farm animals, (e.g. poultry (for example chickens, ducks, geese and turkeys), pigs, cows, goats and sheep). The microbial lipids preferably constitute from 1 to 99% wt. of the animal's dietary lipid intake, more especially 10 to 90% wt. particularly 20 to 80% wt., more particularly 30 to 70% wt. In general, it is preferred that the animal's diet includes further lipids of non-microbial origin, e.g. fish oils or plant oils, to ensure the necessary intake of polyunsaturated fatty acids and C₁₈ fatty acids. Soy bean oil, rape seed oil, sunflower oil, maize oil, evening primrose oil and fish oils are especially preferred for use in this regard.

The use of the microbial lipids in the feed for food animals (e.g. chicken etc.) may also lower the cholesterol in the meat or eggs (avian eggs that is) that are produced from or by such animals for human consumption. Desirably the animal is fed the microbial lipids for a period of at least one week, preferably at least two weeks before the reduced cholesterol food product (e.g. meat or poultry eggs) is harvested.

The microbial lipids (or a microbe-based product such as a homogenizate etc.) may be administered alone; however more generally they will be formulated together with other nutritionally useful materials, e.g. meat, plant oils, fish oils, carbohydrates, flavours, vitamins, minerals, etc. Such a formulated product, since it has a therapeutically or prophylactically desired effect as well as a nutritional effect will generally be referred to as a functional food or as a nutraceutical composition. It is preferred that the microbial lipid constitute from 1 to 99% wt. of such a nutraceutical composition, more preferably 2 to 50% wt., especially 5 to 20% wt. If the nutraceutical composition, as is preferred, is in the form of a total feed (i.e. one which can supply the entire nutritional needs of the animal in terms of protein, carbohydrate and lipids), then it may be given to the animal at frequencies and in quantities routine for food for the size, age, gender and species of the animal in question.

The microbe source of the methanotrophic microbial lipid is preferably a bacterium or a mixture of bacteria cultured using methane as the carbon source, e.g. a *Methylococcus* species such as *M.capsulatus,* optionally together with *Ralstonia* (formerly *Alcaligenes)* and/or *Bacillus* and/or *Aneurinibacillus* species, such as *Ralstonia sp, Aneurinibacillus sp* and *B. agri.* Such strains are available from Norferm Danmark A/S, Stenhuggervej 22, DK-5230 Odense, Denmark. The microbes might be administered as live cultures; however more normally the microbe will be killed before administration (e.g. as a natural consequence of a homogenization or sterilization step, or before lipid extraction).

Quite surprisingly, if microbial lipids are extracted from the microbial biomass, the residual material (which contains proteins, nucleic acids, carbohydrates, etc) is of enhanced digestibility relative to the biomass from which the lipid has not been extracted. The lipid-depleted biomass and lipid-depleted biomass derivatives (e.g. homogenizates) may be used in or as feedstuffs or feedstuff additives.

The invention will now be illustrated with reference to the following non-limiting Examples.

### Example 1

### Mink

The effects on plasma cholesterol in mink (*Mustela vison*) of three different high lipid diets (56E% from lipids, i.e. with lipids providing 56% of dietary energy input) with lipids extracted using methanol and chloroform from BPM (a bacterial biomass) replacing 0%, 17% and 67% of those in soybean oil, were studied. BPM is produced by culturing *Methylococcus capsulatus, Rastonia sp*., *Brevibacillus agri* and *Aneurinibacillus sp*. using natural gas (99% methane), ammonia and mineral salts as described in WO 01/60974 and WO 03/016460. Phospholipids are the main lipid component in BPM, consisting mainly of phosphatidylethanolamines, together with some phosphatidylglycerols, with predominantly 16:0 and 16:1 fatty acids and no polyunsaturated fatty acids.

The 0%, 17% and 67% bacterial lipid diet compositions were as set out in Table 1 below.

**Table 1**

| Ingredient | 0% | 17% | 67% |
|---|---|---|---|
| Corn starch | 62.9 | 62.9 | 62.9 |
| Coalfish fillet | 651.4 | 651.4 | 651.4 |
| Soybean oil | 91.4 | 61 | 15.3 |
| Bacterial lipids | - | 30.5 | 76.2 |
| Sunflower oil | 2.29 | 2.29 | 2.29 |
| Vitamin/mineral mix (100 kCal/100g) | 0.84 | 0.84 | 0.84 |
| BHT (100 mg/kg) | 0.08 | 0.08 | 0.08 |
| Calcium phosphate | 1.63 | 1.63 | 1.63 |
| Calcium carbonate | 1.85 | 1.85 | 1.85 |
| Water | 187.7 | 187.7 | 187.7 |

The figures in columns are in g/kg.

18 growing mink were fed one of the three diets during a 25-day period in a parallel group design. The mink had their main part of the plasma cholesterol in the HDL fraction. Total cholesterol, LDL cholesterol, HDL cholesterol, VLDL cholesterol and the LDL/HDL cholesterol ratio were significantly lower by 34.5%, 49.20, 28.8%, 29.7%, and 26.8%, respectively, when the mink consumed diets with 67% lipids from BPM and 33% lipids from soybean oil as compared to diets containing 100% lipids from soybean oil.

These reductions are especially significant since soybean oil itself is known to have a plasma cholesterol lowering effect.

The plasma concentrations of triacylglycerols (i.e. triglycerides) were also measured and were lower by 31.0% when the mink consumed diets with 67% lipids from BPM and 33% lipids from soybean oil as compared to diets containing 100% lipids from soybean oil.

### Example 2

### Pigs

Pigs were fed BPM as 0, 5, 10 and 15%wt of their diet. BPM contains about 10%wt microbial lipids and thus the highest BPM content diet provided about 25%wt ototal dietary lipids. Plasma from the pigs was analysed for cholesterol content and, while the total cholesterol content was not significantly altered, the proportion of the cholesterol which was HDL cholesterol, ie the cardioprotective form, was increased. The plasma HDL cholesterol levels four the four diets were respectively 1.017, 1.077, 1.073 and 1.227 mol/L.

## Claims

1. Microbial lipids produced by methanotrophic bacteria for oral administration for use in the treatment of animal subjects to reduce plasma cholesterol levels therein, to maintain a reduced plasma cholesterol level therein or to reduce the ratio of LDL to HDL cholesterol in the plasma thereof.

2. Microbial lipids produced by methanotrophic bacteria for oral administration for use in the treatment of animal subjects for improving cardiovascular health by increasing plasma docosahexaenoic acid (DHA).

3. Microbial lipids for use according to claim 1 or claim 2 for administration to a human.

4. Microbial lipids for use according to claim 1 or claim 2 for administration to a fish.

5. Microbial lipids for use according to claim 1 or claim 2 for administration to a juvenile fish.

6. Microbial lipids for use according to any of the preceding claims for administration in encapsulated form.

7. Microbial lipid produced by methanotrophic bacteria for use as a medicament.

8. Methylococcus lipid for use as a medicament.

9. A methanotrophic bacterium-derived phospholipid for use in medicine.

10. A methanotrophic bacterium-derived phospholipid for use according to claim 9, which phospholipid is a phosphatidylethanolamine.

11. A methanotrophic bacterium-derived phospholipid for use according to claim 9, which phospholipid is a phosphatidylethanolamine with C16:0 and/or C16:1 fatty acid side chains.

12. A methanotrophic bacterium-derived phospholipid for use according to any one of claims 9 to 11, which phospholipid is at least 80% wt. pure.

13. A methanotrophic bacterium-derived phospholipid for use according to any one of claims 9 to 11, which phospholipid is at least 80% wt. pure and in a quantity of at least 10g.

14. A foodstuff containing a microbial lipid extract and a further nutrient component, preferably of non-microbial origin, wherein said lipid is produced by methanotrophic bacteria.

15. A pharmaceutical or nutraceutical composition comprising a microbial lipid extract together with a pharmaceutically acceptable carrier or excipient, wherein said lipid is produced by methanotrophic bacteria.

16. The use of microbial lipids produced by methanotrophic bacteria for the manufacture of a medicament for oral administration for use in the treatment of animal subjects to reduce plasma cholesterol levels therein, to maintain a reduced plasma cholesterol level therein or to reduce the ratio of LDL to HDL cholesterol in the plasma thereof.

17. The use of microbial lipids produced by methanotrophic bacteria for the manufacture of a medicament for oral administration for use in the treatment of animal subjects for improving cardiovascular health by increasing plasma docosahexaenoic acid (DHA).

## Patentansprüche

1. Mikrobielle Lipide, die durch methanotrophe Bakterien erzeugt werden, zur oralen Verabreichung zur Verwendung bei der Behandlung von tierischen Subjekten, um Plasmacholesterinspiegel darin zu verringern, um einen verringerten Plasmacholesterinspiegel darin aufrechtzuerhalten oder um das Verhältnis von LDL- zu HDL-Cholesterin im Plasma davon zu verringern.

2. Mikrobielle Lipide, die durch methanotrophe Bakterien erzeugt werden, zur oralen Verabreichung zur Verwendung bei der Behandlung von tierischen Subjekten zum Verbessern der kardiovaskulären Gesundheit durch Erhöhen von Plasmadocosahexaensäure (DHA).

3. Mikrobielle Lipide zur Verwendung nach Anspruch 1 oder Anspruch 2 zur Verabreichung an einen Menschen.

4. Mikrobielle Lipide zur Verwendung nach Anspruch 1 oder Anspruch 2 zur Verabreichung an einen Fisch.

5. Mikrobielle Lipide zur Verwendung nach Anspruch 1 oder Anspruch 2 zur Verabreichung an einen juvenilen Fisch.

6. Mikrobielle Lipide zur Verwendung nach einem der vorhergehenden Ansprüche zur Verabreichung in eingekapselter Form.

7. Mikrobielles Lipid, das durch methanotrophe Bakterien erzeugt wird, zur Verwendung als Medikament.

8. Methylococcuslipid zur Verwendung als Medikament.

9. Von methanotrophen Bakterien abgeleitetes Phospholipid zur Verwendung in einem Arzneimittel.

10. Von methanotrophen Bakterien abgeleitetes Phospholipid zur Verwendung nach Anspruch 9, wobei das Phospholipid ein Phosphatidylethanolamin ist.

11. Von methanotrophen Bakterien abgeleitetes Phospholipid zur Verwendung nach Anspruch 9, wobei das Phospholipid ein Phosphatidylethanolamin mit C16:0- und/oder C16:1-Fettsäure-Seitenketten ist.

12. Von methanotrophen Bakterien abgeleitetes Phospholipid zur Verwendung nach einem der Ansprüche 9 bis 11, wobei das Phospholipid zu mindestens 80 Gew.-% rein ist.

13. Von methanotrophen Bakterien abgeleitetes Phospholipid zur Verwendung nach einem der Ansprüche 9 bis 11, wobei das Phospholipid zu mindestens 80 Gew.-% rein ist und in einer Menge von mindestens 10 g vorliegt.

14. Nahrungsmittel, das einen mikrobiellen Lipidextrakt und eine weitere Nährstoffkomponente, vorzugsweise nicht mikrobiellen Ursprungs, enthält, wobei das Lipid durch methanotrophe Bakterien erzeugt wird.

15. Pharmazeutische oder nutrazeutische Zusammensetzung mit einem mikrobiellen Lipidextrakt zusammen mit einem pharmazeutisch verträglichen Träger oder Exzipienten, wobei das Lipid durch methanotrophe Bakterien erzeugt wird.

16. Verwendung von mikrobiellen Lipiden, die durch methanotrophe Bakterien erzeugt werden, für die Herstellung eines Medikaments zur oralen Verabreichung zur Verwendung bei der Behandlung von tierischen Subjekten, um Plasmacholesterinspiegel darin zu verringern, um einen verringerten Plasmacholesterinspiegel darin aufrechtzuerhalten oder um das Verhältnis von LDL- zu HDL-Cholesterin im Plasma davon zu verringern.

17. Verwendung von mikrobiellen Lipiden, die durch methanotrophe Bakterien erzeugt werden, für die Herstellung eines Medikaments zur oralen Verabreichung zur Verwendung bei der Behandlung von tierischen Subjekten zum Verbessern der kardiovaskulären Gesundheit durch Erhöhen von Plasmadocosahexaensäure (DHA).

## Revendications

1. Lipides microbiens produits par des bactéries méthanotrophes pour une administration orale en vue d'une utilisation dans le traitement de sujets animaux pour réduire des niveaux de cholestérol plasmatique chez ceux-ci, pour maintenir un niveau de cholestérol plasmatique réduit chez ceux-ci ou pour réduire le taux de cholestérol LDL à HDL dans le plasma de ceux-ci.

2. Lipides microbiens produits par des bactéries méthanotrophes pour une administration orale en vue d'une utilisation dans le traitement de sujets animaux pour améliorer la santé cardio-vasculaire en augmentant l'acide docosahexaénoïque (DHA) du plasma.

3. Lipides microbiens destinés à une utilisation selon la revendication 1 ou la revendication 2 en vue d'une administration à un être humain.

4. Lipides microbiens destinés à une utilisation selon la revendication 1 ou la revendication 2 en vue d'une administration à un poisson.

5. Lipides microbiens destinés à une utilisation selon la revendication 1 ou la revendication 2 en vue d'une administration à un poisson juvénile.

6. Lipides microbiens destinés à une utilisation selon l'une quelconque des revendications précédentes en vue d'une administration sous forme encapsulée.

7. Lipides microbiens produits par des bactéries méthanotrophes en vue d'une utilisation comme médicament.

8. Lipide issu de Méthylococcus destiné à une utilisation comme médicament.

9. Phospholipide dérivé d'une bactérie méthanotrophe destiné à une utilisation en médecine.

10. Phospholipide dérivé d'une bactérie méthanotrophe destiné à une utilisation selon la revendication 9, lequel phospholipide est une phosphatidyléthanolamine.

11. Phospholipide dérivé d'une bactérie méthanotrophe destiné à une utilisation selon la revendication 9, lequel phospholipide est une phosphatidyléthanolamine avec des chaînes latérales d'acides gras C16:0 et/ou C16:1.

12. Phospholipide dérivé d'une bactérie méthanotrophe destiné à une utilisation selon l'une quelconque des revendications 9 à 11, lequel phospholipide est pur à au moins 80 % en poids.

13. Phospholipide dérivé d'une bactérie méthanotrophe destiné à une utilisation selon l'une quelconque des revendications 9 à 11, lequel phospholipide est pur à au moins 80 % en poids et dans une quantité d'au moins 10 g.

14. Denrée alimentaire contenant un extrait de lipide microbien et un composant nutritif supplémentaire, de préférence d'origine non microbienne, dans laquelle ledit lipide est produit par des bactéries méthanotrophes.

15. Composition pharmaceutique ou nutraceutique comportant un extrait de lipide microbien associé à un porteur ou un excipient pharmaceutiquement acceptable, dans laquelle ledit lipide est produit par des bactéries méthanotrophes.

16. Utilisation de lipides microbiens produits par des bactéries méthanotrophes pour la fabrication d'un médicament destiné à une administration orale en vue d'une utilisation dans le traitement de sujets animaux pour réduire des niveaux de cholestérol plasmatique chez ceux-ci, pour maintenir un niveau de cholestérol plasmatique réduit chez ceux-ci ou pour réduire le taux de cholestérol LDL à HDL dans le plasma de ceux-ci.

17. Utilisation de lipides microbiens produits par des bactéries méthanotrophes pour la fabrication d'un médicament destiné à une administration orale en vue d'une utilisation dans le traitement de sujets animaux pour améliorer la santé cardio-vasculaire en augmentant l'acide docosahexaénoïque (DHA) du plasma.
